# FASCICULE DE BREVET EUROPEEN

(11) **EP 4 355 251 B1**
(45) Date de publication et mention de la délivrance du brevet: **24.12.2025**
(21) Numéro de dépôt: 22741337.4
(22) Date de dépôt: 14.06.2022
(51) Int. Cl.: A61B 34/30, A61B 90/11, A61B 34/10, A61B 34/20, A61B 90/00

(54) **ROBOT ÉQUIPÉ D'UNE SONDE ÉCHOGRAPHIQUE POUR LE GUIDAGE TEMPS-RÉEL D'INTERVENTIONS PERCUTANÉES**
ROBOTER MIT ULTRASCHALLSONDE ZUR ECHTZEIT-FÜHRUNG BEI PERKUTANEN EINGRIFFEN
ROBOT EQUIPPED WITH AN ULTRASOUND PROBE FOR REAL-TIME GUIDANCE IN PERCUTANEOUS INTERVENTIONS

(30) Priorité: 16.06.2021 FR 2106352
(43) Date de publication de la demande: 24.04.2024
(73) Titulaire: Quantum Surgical, 34000 Montpellier (FR)
(72) Inventeur: SUDRE, Mathieu, 34160 Restinclières (FR); BLONDEL, Lucien, 34070 Montpellier (FR); NAHUM, Bertin, 34170 Castelnau-le-Lez (FR); BADANO, Fernand, 69006 Lyon (FR)
(74) Mandataire: Santarelli
(86) Numéro de dépôt international: PCT/FR2022/051137
(87) Numéro de publication internationale: WO 2022/263764

(56) Documents cités:
- WO-A1-2019/136412
- DE-U1- 202018 104 487
- FR-A1- 3 103 097
- US-A1- 2020 281 667
- US-A1- 2021 113 181
- US-A1- 2021 161 612

## Description

### Domaine de l'invention

La présente demande appartient au domaine des dispositifs robotisés pour assister un praticien lors d'une intervention médicale mini-invasive comprenant l'insertion d'un ou plusieurs instruments médicaux dans une anatomie d'intérêt d'un patient. Notamment, l'invention concerne un robot médical configuré pour suivre le mouvement d'un point cible dans une lésion au sein d'une anatomie d'intérêt d'un patient et pour ajuster en temps réel la position d'un bras articulé du robot pour guider un instrument médical de façon optimale vers le point cible. Le mouvement du point cible peut notamment être engendré par la respiration du patient, ou par l'insertion de l'instrument médical.

### Etat de la technique

Pour préparer une intervention mini-invasive visant à atteindre avec un instrument médical une zone anatomique cible dans une anatomie d'intérêt d'un patient, un praticien effectue généralement une planification de l'intervention à partir d'une image médicale préopératoire (quelques jours ou quelques semaines avant l'intervention) ou pré-interventionnelle (juste avant l'intervention lorsque le patient est installé sur la table d'intervention). L'intervention médicale mini-invasive peut notamment viser à réaliser la biopsie ou l'ablation d'une tumeur dans un organe, à effectuer une vertébroplastie, une cimentoplastie ou encore à stimuler une zone anatomique particulière. L'anatomie d'intérêt peut correspondre par exemple à un poumon, un rein, le foie, le cerveau, le tibia, le genou, une vertèbre, etc. L'instrument médical peut être une aiguille, une sonde, un cathéter, etc.

Au cours de cette étape de planification, le praticien définit un point cible dans une région à traiter de l'anatomie d'intérêt. Le praticien définit également un point d'entrée pour l'instrument médical sur la peau du patient. Ces deux points définissent alors une trajectoire que l'instrument médical doit suivre pour réaliser l'intervention médicale. Dans le cas particulier des organes mous situés dans la région thoracique, la région abdominale ou la région pelvienne, les mouvements liés à la respiration du patient et/ou les déformations locales de l'organe dues à l'insertion de l'instrument médical entrainent un déplacement du point cible pendant l'intervention. Les images médicales de planification préopératoire ou pré-interventionnelle ne permettent pas de prévoir ce déplacement du point cible pendant l'intervention. Ainsi, la position du point cible (c'est-à-dire la position de la région à traiter dans l'anatomie d'intérêt) est généralement différente lors de l'acquisition de l'image médicale de planification et lors de l'intervention. Par conséquent, lorsque l'insertion de l'instrument médical est planifiée à partir de l'image médicale de planification, il y a un risque que le point cible ne soit pas atteint avec précision par l'instrument médical.

En outre, il existe un risque que l'instrument médical se courbe au cours de l'insertion et qu'il n'atteigne pas le point cible si la trajectoire planifiée que doit suivre l'instrument médical n'est pas ajustée en conséquence.

Pour limiter le déplacement du point cible engendré par la respiration du patient, il est envisageable, au moment de l'insertion de l'instrument médical, de bloquer la respiration du patient à une phase du cycle respiratoire correspondant à celle à laquelle l'image médicale de planification a été acquise. Le blocage de la respiration peut être effectué de façon volontaire par le patient si l'intervention médicale a lieu sous anesthésie locale, ou bien de façon contrôlée par le praticien si l'intervention médicale a lieu sous anesthésie générale (interruption de la ventilation mécanique). Cette solution n'est cependant pas toujours très précise car il est difficile d'obtenir une correspondance exacte entre la phase du cycle respiratoire à laquelle l'image médicale de planification a été acquise et la phase du cycle respiratoire à laquelle la respiration du patient est bloquée pendant l'intervention. En outre, cette solution suppose une insertion relativement rapide de l'instrument médical puisque celle-ci doit se faire pendant que la respiration du patient est bloquée.

Il est également envisageable de prendre plusieurs images médicales de planification au cours d'un cycle respiratoire du patient et de déterminer la trajectoire la moins soumise aux déformations et aux déplacements de l'anatomie d'intérêt engendrés par la respiration. Toutefois, il y a là encore un risque que le point cible ne soit pas atteint avec précision par l'instrument médical.

Il est également envisageable de suivre la position du point cible tout au long de l'intervention en faisant régulièrement l'acquisition d'images médicales intra-interventionnelles (images acquises lorsque l'instrument médical est inséré dans le corps du patient). Ces images médicales sont généralement acquises par tomodensitométrie, par rayons X ou par résonance magnétique. Dans le cas de la tomodensitométrie ou des rayons X, une telle solution présente toutefois l'inconvénient d'irradier significativement le patient et le praticien pendant l'intervention. Dans le cas de l'imagerie par résonance magnétique, il est nécessaire d'utiliser du matériel spécifique amagnétique, notamment pour le matériel anesthésique, ce qui est particulièrement contraignant. Cette solution suppose en outre l'utilisation de dispositifs d'imagerie encombrants tout au long de l'intervention.

Il est également connu de suivre la position d'une lésion au sein d'une anatomie d'intérêt à l'aide d'images échographiques. Toutefois, la lésion n'est pas toujours visible sur une image échographique, et les solutions existantes manquent généralement de précision.

Il reste donc nécessaire de trouver une solution pour insérer un instrument médical avec précision au niveau d'un point cible d'une région à traiter au sein d'une anatomie d'intérêt d'un patient, notamment lorsque les mouvements liés à la respiration du patient et/ou les déformations locales de l'anatomie d'intérêt dues à l'insertion de l'instrument médical entrainent un déplacement du point cible pendant l'intervention.

La demande de brevet FR3103097A1 concerne un système de navigation optique pour déterminer la position d'une anatomie d'intérêt d'un patient.

La demande de brevet DE202018104487U1 décrit un système de biopsie comprenant un bras robotisé, un dispositif de commande du bras robotisé, et un capteur à ultrasons fixé sur une bride du bras robotisé.

La demande de brevet US2021/161612A1 décrit un système de positionnement d'un guide-aiguille par ultrasons. Le système comporte un écran de réalité augmentée tel qu'un casque porté par un utilisateur.

La demande de brevet US2021/113181A1 décrit un système de balayage à ultrasons comprenant un bras robotisé avec une caméra, une sonde à ultrasons montée à une extrémité du bras robotisé, un capteur de force et un ordinateur hôte.

La demande de brevet US2020/281667A1 concerne un dispositif robotique permettant d'effectuer une intervention médicale sur un patient à l'aide d'un instrument médical. Le système comporte un bras robotisé équipé d'un instrument médical. La position de l'instrument médical est contrôlée en fonction d'un modèle biomécanique, d'informations de position concernant l'anatomie du patient, et d'une trajectoire à suivre par l'instrument médical pour effectuer l'intervention médicale.

### Exposé de l'invention

Les solutions proposées dans la présente demande ont pour objectif de remédier à tout ou partie des inconvénients de l'art antérieur, notamment ceux exposés ci-avant.

A cet effet, et selon un premier aspect, il est notamment proposé un robot médical pour assister un praticien lors d'une intervention médicale pour traiter une lésion dans une anatomie d'intérêt d'un patient. Le robot médical comporte un bras robotisé auquel est fixé à une extrémité une sonde à ultrasons et un guide-outil destiné à guider un instrument médical. Le robot médical comporte également une unité de contrôle configurée pour contrôler le bras robotisé. Le robot médical étant configuré pour coopérer avec un système de navigation. L'unité de contrôle est configurée pour pouvoir déterminer à tout instant, à partir d'informations communiquées par le système de navigation, la position d'un marqueur robot destiné à être positionné sur le robot médical et la position d'un marqueur patient destiné à être positionné sur le patient à proximité de l'anatomie d'intérêt. Pendant une phase de préparation, l'unité de contrôle est configurée pour :
- recevoir une image de planification sur laquelle est visible la lésion et au moins un élément radio-opaque du marqueur patient,
- déterminer à partir de l'image de planification un point cible au niveau de la lésion et un point d'entrée au niveau de la peau du patient, le point cible et le point d'entrée définissant ainsi une trajectoire à suivre pour l'instrument médical,
- contrôler le bras robotisé, en fonction de la position du marqueur robot et de la position du marqueur patient, pour placer la sonde à ultrasons au contact du patient et dans un plan contenant la lésion et la trajectoire à suivre.
Pendant une phase de guidage, l'unité de contrôle est configurée pour recevoir en temps réel des images échographiques acquises par la sonde à ultrasons et pour contrôler en temps réel le bras robotisé, à partir desdites images échographiques, afin de placer le guide-outil de sorte à guider l'instrument médical selon la trajectoire à suivre. En outre, l'unité de contrôle est en outre configurée pour comparer une image échographique avec l'image de planification, et pour déterminer une direction dans laquelle il convient de déplacer la sonde à ultrasons pour qu'une image échographique acquise par la sonde à ultrasons comporte une région anatomique dans laquelle se trouve la lésion.

Dans la présente demande, le terme « position » doit être entendu au sens large comme décrivant à la fois la position et l'orientation d'un objet dans un repère tridimensionnel (le terme « pose » est parfois utilisé dans la littérature anglo-saxonne). Les positions des marqueurs (marqueur patient et marqueur robot) ainsi que la position du point cible et la position du point d'entrée peuvent être définies dans un référentiel du robot ou dans un référentiel du système de navigation. Il convient de noter que le référentiel du robot peut être défini relativement au référentiel du système de navigation car la position du marqueur robot est connue à la fois dans le référentiel du système de navigation et dans le référentiel du robot (chaque articulation du bras robotisé comporte par exemple un codeur permettant de connaître la position de chaque élément articulé du bras robotisé dans le référentiel du robot, et la position du marqueur robot sur le robot est connue a priori par l'unité de contrôle).

L'image de planification est par exemple une image médicale pré-interventionnelle acquise juste avant l'intervention lorsque le patient est installé sur la table d'intervention, à un moment où le marqueur patient est positionné sur le patient à proximité de l'anatomie d'intérêt. L'image de planification peut également être une image médicale préopératoire acquise quelques jours ou quelques semaines avant l'intervention et recalée avec une image pré-interventionnelle. L'image de planification est par exemple une image médicale de tomodensitométrie, de tomographie par émission de positons ou d'imagerie par résonance magnétique. La position du marqueur patient peut être déterminée sur l'image de planification grâce au marqueur radio-opaque du marqueur patient qui est visible sur l'image de planification.

Le point cible et le point d'entrée peuvent être déterminés sur l'image de planification par un algorithme d'intelligence artificielle de segmentation. Selon un autre exemple, le point cible et le point d'entrée peuvent être déterminés sur l'image de planification par le praticien.

Le point cible et le point d'entrée initialement définis par le praticien sur l'image de planification peuvent ensuite être suivis pendant la phase de guidage sur les images échographiques acquises par la sonde à ultrasons (par exemple par un algorithme de suivi de la déformation du « speckle » (le « speckle » représente l'ensemble des petites tâches rapidement fluctuantes qui apparaissent dans la texture instantanée d'une image et qui lui donnent un aspect granuleux).

Il est ainsi possible de suivre en temps réel la position du point cible et la position du point d'entrée à partir des images échographiques. Le bras robotisé peut alors être déplacé en temps réel pour qu'il soit en permanence positionné de telle sorte que l'instrument médical soit guidé selon la trajectoire définie par la position du point cible et la position du point d'entrée. Cet ajustement en temps réel de la position du bras robotisé permet de compenser le mouvement du point cible engendré par la respiration du patient. Ce suivi en temps réel peut notamment avoir lieu pendant une phase de guidage du guide-outil, juste avant l'insertion de l'instrument médical.

Avec de telles dispositions, il devient possible de bloquer la respiration du patient à n'importe quel instant du cycle respiratoire pour procéder à l'insertion de l'instrument médical. En effet, quel que soit l'instant où la respiration du patient est bloquée, le bras robotisé sera correctement positionné pour permettre l'insertion de l'instrument médical selon la trajectoire souhaitée.

Aussi, il n'est même plus indispensable de bloquer la respiration du patient pendant l'intervention. En effet, le bras robotisé est déplacé en temps réel pour que la position du bras robotisé soit constamment ajustée pour guider l'instrument médical selon la trajectoire souhaitée.

L'invention permet en outre de minimiser les réajustements latéraux de la trajectoire après l'insertion de l'instrument médical (de tels réajustements latéraux de la trajectoire sont généralement traumatiques pour l'organe traversé par l'instrument médical).

L'instrument médical peut ainsi être inséré au niveau de la région à traiter avec une très grande précision, quel que soit l'instant d'insertion de l'instrument médical au cours du cycle respiratoire. L'insertion de l'instrument médical est généralement mise en œuvre par le praticien, le robot médical ayant pour but de guider l'insertion de l'instrument médical par le praticien. Rien n'empêcherait toutefois que l'insertion de l'instrument médical soit automatisée et contrôlée par l'unité de contrôle.

En outre, comme la détermination en temps réel de la position du point cible et de la position du point d'entrée pendant l'intervention est réalisée à partir d'images échographiques, le patient et le praticien ne sont pas exposés à des rayonnements ionisants au cours de l'intervention.

Dès que l'instrument médical commence à être inséré dans le corps du patient, la position du point d'entrée au niveau de la peau du patient est fixe et devient un pivot de rotation pour les mouvements du bras robot. Il reste toutefois possible de suivre en temps réel la position du point cible, la position du point d'entrée à partir de nouvelles images échographiques acquises en temps réel pendant l'insertion de l'instrument médical.

De telles dispositions permettent de prendre en compte un éventuel déplacement du point cible résultant de l'insertion de l'instrument médical. Le point cible peut en effet se déplacer dans la direction de la trajectoire suivie par l'instrument médical au cours de son insertion (c'est notamment le cas lorsque le point cible à atteindre est dans une lésion, par exemple une tumeur, au sein d'un organe mou). La détermination en temps réel de la position du point cible à l'aide des images échographiques permettent de mettre à jour en temps réel la trajectoire que doit suivre l'instrument médical ainsi que la position du bras robotisé pour guider l'instrument médical selon cette trajectoire.

Dans des modes particuliers de réalisation, l'invention peut comporter en outre l'une ou plusieurs des caractéristiques suivantes, prises isolément ou selon toutes les combinaisons techniquement possibles.

Dans des modes particuliers de réalisation, pendant la phase de guidage, l'unité de contrôle est configurée, pour chaque image échographique reçue, pour :
- générer une image de fusion résultant d'un recalage de l'image échographique avec l'image de planification,
- déterminer la position du point cible et la position du point d'entrée à partir de l'image de fusion,
- déplacer le bras robotisé de sorte que l'instrument médical soit guidé par le guide-outil selon la trajectoire définie par la position du point cible et la position du point d'entrée.

Le résultat du recalage d'une image échographique avec l'image de planification produit une image de fusion sur laquelle la lésion est visible. Le point cible et le point d'entrée initialement déterminées sur l'image de planification peuvent alors aussi être identifiés sur l'image de fusion.

Dans des modes particuliers de réalisation, pendant la phase de guidage, lors de l'insertion de l'instrument médical, et pour chaque nouvelle image échographique reçue, l'unité de contrôle est configurée pour déterminer la position de l'instrument médical et pour ajuster le contrôle en temps réel du bras robotisé en fonction de la position de l'instrument médical.

De telles dispositions permettent de maintenir la sonde à ultrasons au contact du corps du patient avec une pression adéquate pendant les mouvements respiratoires du patient.

Dans des modes particuliers de réalisation, la sonde à ultrasons est couplée à un capteur d'effort permettant à l'unité de contrôle de déterminer une pression exercée par la sonde à ultrasons sur le corps du patient. L'unité de contrôle est en outre configurée pour déplacer le bras robotisé de sorte que la sonde à ultrasons exerce une pression prédéterminée sur le corps du patient.

De telles dispositions permettent de prendre en compte le risque que l'instrument médical se courbe au cours de l'insertion et d'ajuster le contrôle en temps réel du bras robotisé en conséquence (la trajectoire à suivre par l'instrument médical n'est alors plus une ligne droite entre le point d'entrée et le point cible).

Dans des modes particuliers de réalisation, l'image de planification est une image de tomodensitométrie, une image de tomographie par émission de positons ou une image d'imagerie par résonance magnétique.

Dans des modes particuliers de réalisation, pour déterminer à partir de l'image de planification un point cible et un point d'entrée, l'unité de contrôle est configurée pour segmenter sur l'image de planification la lésion et/ou des zones anatomiques à éviter à l'aide d'un algorithme d'intelligence artificielle.

Dans des modes particuliers de réalisation, les images échographiques reçues de la sonde à ultrasons sont des images échographiques en mode B.

Dans des modes particuliers de réalisation, l'unité de contrôle est configurée pour recevoir et traiter des images échographiques acquises par la sonde à ultrasons à une fréquence au moins égale à quinze images par seconde.

De telles dispositions permettent de garantir un suivi en temps réel de la position du point cible et par conséquent un ajustement en temps réel de la position du bras robotisé pour que l'instrument médical soit guidé selon la trajectoire souhaitée tout au long de l'intervention.

Dans des modes particuliers de réalisation, le robot médical comporte en outre une interface utilisateur comprenant un écran d'affichage permettant au praticien de visualiser l'image de planification et/ou les images de fusion.

Dans des modes particuliers de réalisation, l'interface utilisateur comporte des moyens d'entrée permettant au praticien, pendant la phase de préparation, d'identifier sur l'image de planification affichée sur l'écran d'affichage un point cible et/ou un point d'entrée et/ou une zone anatomique qui ne doit pas être traversée par l'instrument médical.

Dans des modes particuliers de réalisation, l'interface utilisateur comprend un dispositif de réalité augmentée permettant de superposer les images d'analyse avec des images réelles du corps du patient sur l'écran d'affichage.

Le dispositif de réalité augmentée permet de superposer au corps du patient la lésion en mouvement et en trois dimensions ainsi que la progression de l'instrument médical au cours de son insertion. Il peut s'agir par exemple d'un écran positionné sur la table d'intervention au-dessus du patient, ou encore d'un masque, d'un casque ou de lunettes de réalité augmentée. Ce type d'affichage facilite la représentation spatiale de l'anatomie d'intérêt du patient par le praticien.

### Présentation des figures

L'invention sera mieux comprise à la lecture de la description suivante, donnée à titre d'exemple nullement limitatif, et faite en se référant aux figures 1 à 6 qui représentent :
[Fig. 1] une représentation schématique d'un dispositif médical comportant un robot médical selon l'invention et un système de navigation,
[Fig. 2] une représentation schématique du bras robotisé du robot médical,
[Fig. 3] une représentation schématique d'un « marqueur robot » destiné à être fixé sur le robot médical,
[Fig. 4] une représentation schématique d'un « marqueur patient » destiné à être positionné sur le patient à proximité de l'anatomie d'intérêt,
[Fig. 5] une représentation schématique d'étapes mises en œuvre par l'unité de contrôle pendant une phase de préparation puis pendant une phase de guidage en temps réel du bras robotisé,
[Fig. 6] une représentation schématique d'une image de planification (partie a) de la figure), une image échographique (partie b) de la figure), et une image de fusion résultant du recalage de l'image de planification et de l'image échographique (partie c) de la figure).

Dans ces figures, des références identiques d'une figure à une autre désignent des éléments identiques ou analogues. Pour des raisons de clarté, les éléments représentés ne sont pas nécessairement à une même échelle, sauf mention contraire.

### Description détaillée d'un mode de réalisation de l'invention

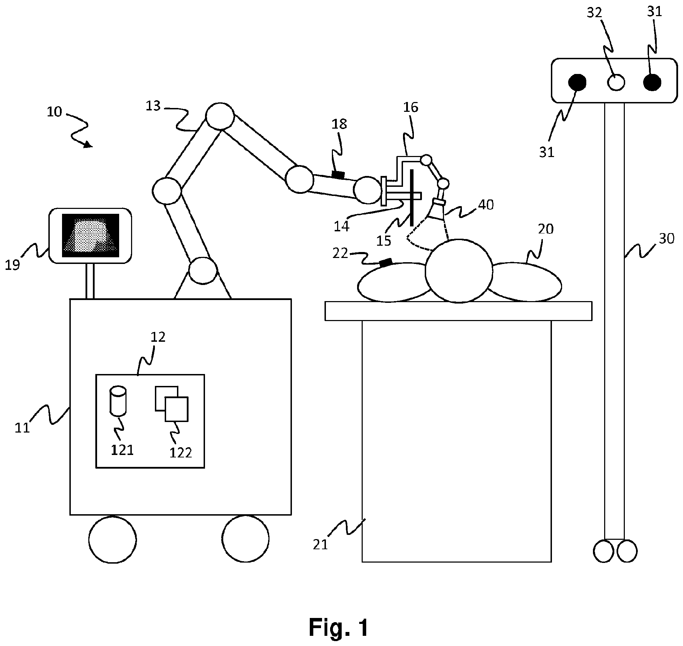

La figure 1 représente schématiquement un robot médical 10 selon l'invention. Le robot médical 10 est utilisé pour assister un praticien lors d'une intervention médicale sur une anatomie d'intérêt d'un patient 20 positionné sur une table d'intervention 21.

On se place à titre d'exemple dans le cas d'une intervention médicale effectuée par voie mini-invasive ou percutanée pour traiter une lésion au sein de l'anatomie d'intérêt du patient. Ce genre d'intervention nécessite généralement l'insertion par le praticien d'un ou plusieurs instruments médicaux (par exemple une aiguille, une sonde, un cathéter, etc.) dans le corps du patient jusqu'à une certaine profondeur pour atteindre une zone anatomique cible (une lésion, par exemple une tumeur) dans l'anatomie d'intérêt (par exemple dans le foie, un poumon, un rein, etc.).

Le robot médical 10 comporte une base 11. Dans l'exemple considéré, la base 11 du robot médical 10 est équipée de roues motorisées, ce qui permet au robot médical 10 de se déplacer selon différentes directions par des mouvements de translation et/ou de rotation.

Le robot médical 10 comporte en outre un bras robotisé 13 articulé dont une extrémité est reliée à la base 11. A l'autre extrémité du bras robotisé 13 sont fixés une sonde à ultrasons 40 et un guide-outil 14 destiné à guider un instrument médical 15, comme par exemple une aiguille, une sonde, un cathéter, une électrode, etc.

Dans l'exemple considéré et illustré sur la figure 1, la sonde à ultrasons 40 est fixée au bras robotisé 13 via un bras supplémentaire 16. Le bras supplémentaire 16 est également articulé afin de laisser au moins un degré de liberté supplémentaire à la sonde à ultrasons 40 par rapport au guide-outil 14.

Le robot médical 10 comporte une unité de contrôle 12 configurée pour contrôler le déplacement du bras robotisé 13. Dans la présente demande, il est considéré que le contrôle du bras robotisé 13 inclut également le contrôle du bras supplémentaire 16. L'unité de contrôle 12 comporte un ou plusieurs processeurs 122 et une mémoire 121 (disque dur magnétique, mémoire électronique, disque optique, etc.) dans laquelle est mémorisée un produit programme d'ordinateur, sous la forme d'un ensemble d'instructions de code de programme à exécuter pour mettre en œuvre les différentes étapes d'un procédé de positionnement du bras robotisé 13. La mémoire 121 permet également d'enregistrer les images et autres informations (notamment les informations de navigation) utilisées pour mettre en œuvre ce procédé.

Le robot médical 10 peut alors être utilisé pour aider un praticien à positionner, maintenir, et guider l'instrument médical 15 au cours de l'intervention médicale. Dans une variante, l'insertion de l'instrument médical 15 peut être entièrement automatisée et contrôlée par l'unité de contrôle 12 du robot médical 10. En outre, le robot médical 10 est utilisé pour positionner automatiquement la sonde à ultrasons 40.

Le robot médical 10 peut également comporter une interface utilisateur 19 comprenant un écran d'affichage permettant au praticien de visualiser des images médicales (par exemple une image de planification et/ou des images échographiques acquises par la sonde à ultrasons 40 et fusionnées avec l'image de planification). L'interface utilisateur peut également comporter des moyens d'entrée (clavier, souris, écran tactile, etc.) permettant au praticien d'identifier sur une image de planification affichée sur l'écran d'affichage un point cible et/ou un point d'entrée et/ou une zone anatomique qui ne doit pas être traversée par l'instrument médical 15.

Dans des modes particuliers de réalisation, l'interface utilisateur peut comprendre un dispositif de réalité augmentée permettant de superposer les images fusionnées avec des images réelles du corps du patient sur l'écran d'affichage. Un tel dispositif facilite la représentation spatiale de l'anatomie d'intérêt pour le praticien.

Le robot médical 10 est configuré pour coopérer avec un système de navigation 30. Le robot médical 10 comporte un module de communication connecté à l'unité de contrôle 12 pour échanger des données avec le système de navigation 30. Le système de navigation 30 comporte également un module de communication pour échanger des données avec l'unité de contrôle 12 du robot médical 10. Les communications établies entre l'unité de contrôle 12 et le système de navigation 30 peuvent être des communications filaires ou des communications sans fil. Par souci de simplification, les modules de communication ne sont pas représentés sur la figure 1.

Dans l'exemple considéré, le système de navigation 30 est un système de navigation optique. Le système de navigation 30 comporte deux capteurs optiques 31 correspondant à deux capteurs d'une caméra stéréoscopique fonctionnant dans le domaine des rayonnements infrarouges. Dans l'exemple considéré, le système de navigation 30 comporte en outre une caméra 32 fonctionnant dans le domaine de la lumière visible.

L'unité de contrôle 12 est configurée pour pouvoir déterminer en tout instant, à partir d'informations communiquées par le système de navigation 30, la position d'un marqueur robot 18 destiné à être fixé sur le robot médical 10 et la position d'un marqueur patient 22 destiné à être positionné sur le patient 20 à proximité de l'anatomie d'intérêt.

Dans la présente demande, le terme « position » correspond à la combinaison de la position et de l'orientation d'un objet dans un repère donné qui est généralement un système de coordonnées en trois dimensions. Le terme « pose » est employé dans la littérature anglo-saxonne pour représenter cette combinaison de la position et de l'orientation d'un objet dans l'espace.

L'unité de contrôle 12 est configurée pour recevoir des images échographiques acquises par la sonde à ultrasons 40.

Les images échographiques reçues en provenance de la sonde à ultrasons 40 et les informations reçues en provenance du système de navigation 30 sont synchronisées temporellement par l'unité de contrôle 12 pour pouvoir corréler à un instant donné la position de la lésion avec la position du marqueur patient 22.

De façon conventionnelle, la sonde à ultrasons 40 comprend un ou plusieurs éléments émetteur-récepteur d'ondes sonores (matériaux piézo-électriques, transducteurs électroniques capacitifs). La sonde à ultrasons produit des ondes ultrasonores par effet piézo-électrique indirect. Chaque fois qu'une onde rencontre une structure anatomique, une partie de cette onde revient par réflexion ou diffusion (« speckle ») sous la forme d'un écho. Cet écho est ensuite transformé en courant électrique par effet piézo-électrique direct puis reconstruit en image. La reconstruction d'une image échographique dépend principalement du nombre, de la taille et des positions des éléments émetteur-récepteur de la sonde (résolution latérale et longitudinale), de la durée des pulses d'émission et des temps d'écho (résolution axiale et/ou en profondeur). L'énergie de l'écho réceptionnée est ensuite codée en niveau de gris. Plus l'énergie est haute et plus la portion d'image correspondante (pixel) est blanche. Ce codage en nuance de gris est appelé la « brillance » et le mode échographique associé est appelé « mode-B ». Les images produites par la sonde à ultrasons 40 peuvent être des images en deux dimensions ou des images en trois dimensions. De préférence, la sonde à ultrasons 40 est capable de générer des images à une fréquence au moins égale à quinze images par seconde.

Le mode-B est particulièrement bien adapté lorsque l'anatomie d'intérêt est le foie. Il convient toutefois de noter que l'invention pourrait également s'appliquer avec d'autres modes échographiques, comme par exemple l'élastographie.

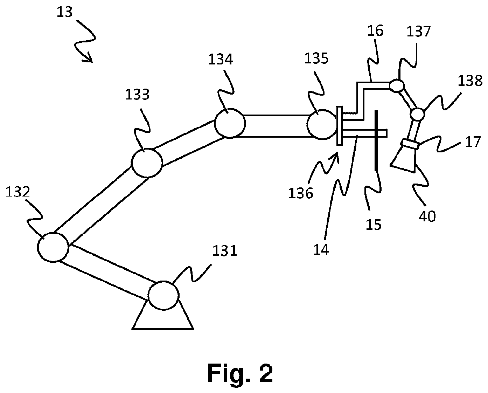

Dans l'exemple considéré, et tel qu'illustré sur la figure 2, le bras robotisé 13 comporte six articulations131 à 136 rotoïdes conférant six degrés de liberté permettant de placer l'instrument médical 15 dans n'importe quelle position de l'espace tridimensionnel. Avantageusement, les articulations 131 à 135 du bras robotisé 13 ne sont pas alignées et présentent un décalage les unes par rapport aux autres, ce qui permet un plus grand nombre de configurations possibles du bras robotisé 13. L'articulation rotoïde 136 correspond à une rotation autour d'un axe parallèle à l'axe principal du guide-outil 14.

Dans l'exemple considéré, la sonde à ultrasons 40 est fixée sur le bras robotisé 13 via un bras supplémentaire 16 comportant deux articulations rotoïdes 137 et 138 conférant deux degrés de liberté supplémentaires pour le déplacement de la sonde à ultrasons 40 par rapport au guide-outil 14. Il convient toutefois de noter que l'inverse est également possible : la sonde à ultrasons 40 pourrait être fixée directement à l'extrémité distale du bras robotisé 13, et un bras supplémentaire pourrait porter le guide-outil 14. Le guide-outil 14 et le bras supplémentaire 16 sont fixés à l'extrémité du bras robotisé 13 par l'intermédiaire d'une bride. Dans l'exemple considéré et illustré à la figure 2, la sonde à ultrasons 40 est en outre couplée à un capteur d'effort 17 permettant à l'unité de contrôle 12 de déterminer une force exercée par le corps du patient 20 sur la sonde à ultrasons 40.

Le bras supplémentaire 16 et le guide-outil 14 sont agencés l'un par rapport à l'autre de telle sorte que l'instrument médical 15 se trouve toujours dans le plan d'une image échographique acquise par la sonde à ultrasons 40.

Chaque articulation 131 à 138 comprend au moins un codeur permettant de connaître en temps réel sa position angulaire. Une configuration du bras robotisé 13 correspond alors à un ensemble de valeurs de paramètres prises par les articulations 131 à 138 (par exemple la valeur d'un angle de rotation pour chaque articulation).

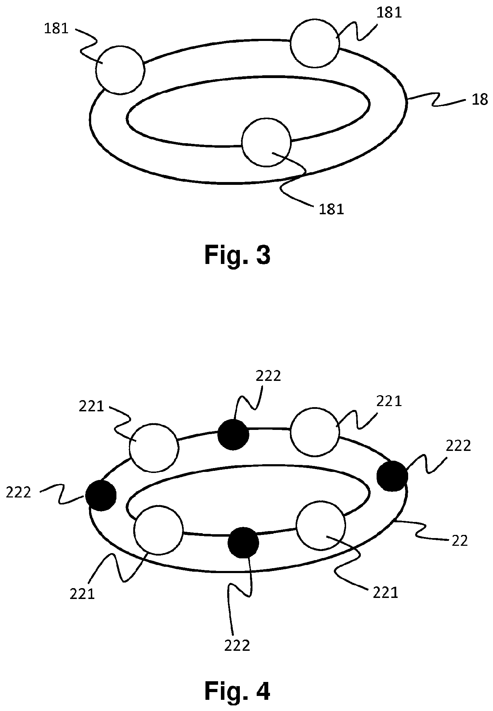

La figure 3 représente schématiquement le marqueur robot 18 destiné à être positionné sur le robot médical 10. Dans l'exemple considéré, le marqueur robot 10 comporte trois marqueurs optiques 181, de telle sorte que la position du marqueur robot 18 puisse être déterminée dans les trois dimensions spatiales du référentiel du système de navigation 30. Les positions respectives des marqueurs optiques 181 du marqueur robot 18 les uns par rapport aux autres sont connues a priori par le système de navigation 30 et/ou par l'unité de contrôle 12. Avantageusement, la forme géométrique de chaque marqueur optique 181 peut également être connue a priori. Dans l'exemple illustré à la figure 3, les marqueurs optiques 181 sont de forme sphérique. La forme sphérique permet d'optimiser la réflexion du rayonnement optique.

L'utilisation d'au moins trois marqueurs optiques 181 permet de définir un plan et donc un repère tridimensionnel orthonormé direct avec un axe z normal au plan et des axes x et y dans le plan de façon que le repère soit direct. Cela permet ainsi de déterminer la position et l'orientation du repère formé à partir des marqueurs optiques 181. Les trois axes x, y et z permettent de définir six degrés de liberté, à savoir une translation selon chacun des axes x, y ou z et une rotation autour de chacun de ces axes.

Les marqueurs optiques 181 peuvent être passifs ou actifs. Des marqueurs optiques passifs réfléchissent un rayonnement optique émis par un autre élément, comme par exemple le système de navigation 30. Des marqueurs optiques passifs peuvent correspondre par exemple à des sphères réfléchissantes détectables par une caméra stéréoscopique infrarouge (c'est ce qui est utilisé par exemple dans les systèmes de navigation Polaris^{®} fabriqués par la société Northern Digital Inc.), ou à des motifs noir et blanc visibles par une caméra stéréoscopique (c'est ce qui est utilisé par exemple dans le système de navigation MicronTracker^{®} de la société ClaroNav). Des marqueurs optiques actifs émettent eux-mêmes un rayonnement optique, par exemple un rayonnement infrarouge, détectable par le système de navigation 30.

Il convient toutefois de noter qu'un seul marqueur optique présentant une forme géométrique caractéristique en trois dimensions pourrait être utilisé à la place de l'ensemble des marqueurs optiques 181 sphériques.

La figure 4 représente schématiquement le marqueur patient 22 destiné à être positionné sur le patient 20 à proximité de l'anatomie d'intérêt. Le marqueur patient 22 comporte au moins trois marqueurs optiques 221 (il en contient quatre dans l'exemple illustré à la figure 4), de telle sorte que la position du marqueur patient 22 puisse être déterminée dans les trois dimensions spatiales du référentiel du système de navigation 30. Les positions respectives des marqueurs optiques 221 du marqueur patient 22 les uns par rapport aux autres sont connues a priori par le système de navigation 30 et/ou par l'unité de contrôle 12. Avantageusement, la forme géométrique de chaque marqueur optique 221 peut également être connue a priori. Dans l'exemple illustré à la figure 4, les marqueurs optiques 221 sont de forme sphérique. La forme sphérique permet d'optimiser la réflexion du rayonnement optique. Ce qui a été mentionné précédemment pour le type actif ou passif des marqueurs optiques 181 du guide-outil 14 est également vrai pour les marqueurs optiques 221 de la référence patient 22. Là encore, il serait envisageable d'utiliser un seul marqueur optique présentant une forme géométrique caractéristique en trois dimensions à la place de l'ensemble des marqueurs optiques 221 sphériques.

Le marqueur patient 22 comporte également des marqueurs radio-opaques 222 qui sont visibles sur une image médicale acquise par un dispositif d'imagerie médicale (par exemple par tomodensitométrie, par résonance magnétique, par ultrasons, par tomographie, par émission de positons, etc.). Les positions respectives des marqueurs radio-opaques 222 les uns par rapport aux autres sont connues a priori par le système de navigation 30 et/ou par l'unité de contrôle 12. Avantageusement, la forme géométrique des marqueurs radio-opaques 222 peut également être connue a priori. De préférence, le marqueur patient 22 comporte au moins trois marqueurs radio-opaques 222 (dans l'exemple considéré, le marqueur patient 22 comporte quatre marqueurs radio-opaques 222). Les marqueurs radio-opaques 222 peuvent être par exemple des billes en céramique. Il convient toutefois de noter qu'un unique marqueur radio-opaque présentant une forme géométrique caractéristique en trois dimensions pourrait être utilisé à la place de l'ensemble des marqueurs radio-opaques 222 sphériques.

Dans la suite de la description, on considère à titre d'exemple nullement limitatif que les capteurs optiques 31 du système de navigation 30 et les différents marqueurs optiques 181, 221 sont conçus pour fonctionner avec un rayonnement optique de type infrarouge. On considère en outre que les marqueurs optiques 181, 221 sont des marqueurs passifs. Les capteurs optiques 31 sont configurés pour émettre un rayonnement infrarouge. Ce rayonnement infrarouge est réfléchi par les différents marqueurs optiques 181, 221 vers les capteurs optiques 31. Les capteurs optiques 31 sont configurés pour recevoir ce rayonnement infrarouge réfléchi. Le système de navigation 30 peut alors déterminer la distance entre un marqueur optique 181, 221 et un capteur optique 31 en mesurant le temps pris par un rayon infrarouge pour faire le trajet aller-retour entre ledit capteur optique 31 et ledit marqueur optique 181, 221. En connaissant la distance entre chaque marqueur optique 181, 221 et chaque capteur optique 31, et en connaissant a priori l'agencement des marqueurs optiques 181, 221 les uns par rapport aux autres sur le marqueur robot 18 et sur le marqueur patient 22, il est possible de déterminer la position du marqueur robot 18 et la position du marqueur patient 22 dans le référentiel du système de navigation 30.

Il convient de noter que l'invention est décrite en utilisant un système de navigation optique. Rien n'empêcherait toutefois d'utiliser, dans une variante, un système de navigation électromagnétique à la place du système de navigation optique. Dans ce cas, les différents « marqueurs » détectables par le système de navigation (marqueur patient 22, marqueur robot 18) correspondraient alors à des capteurs électromagnétiques dont la position peut être déterminée par le système de navigation dans un champ électromagnétique généré.

Dans l'exemple considéré, l'unité de contrôle 12 du robot médical 10 est configuré pour recevoir du système de navigation 30 des informations sur la position courante du marqueur robot 18 dans le référentiel du système de navigation 30. Or, l'unité de contrôle 12 du robot médical 10 connaît la position courante du marqueur robot 18 dans le référentiel du robot médical 10 (via les codeurs des articulations 131 à 138). L'unité de contrôle 12 peut donc déterminer la transformation à opérer pour définir une position dans le référentiel du robot médical 10 à partir d'une position dans le référentiel du système de navigation 30.

Il est en outre possible de déduire la position de la sonde 40 à ultrasons et la position du guide-outil 14 à partir de la position du marqueur robot 18 (via les codeurs des articulations 131 à 138).

L'unité de contrôle 12 est également configurée pour recevoir du système de navigation 30 des informations sur la position du marqueur patient 22 dans le référentiel du système de navigation 30. L'unité de contrôle 10 peut alors définir la position du marqueur patient 22 dans le référentiel du robot médical 10.

La position d'un point d'entrée de l'instrument médical 15 au niveau de la peau du patient et la position d'un point cible au niveau de la lésion à traiter peuvent être déterminées relativement à la position du marqueur patient 22 sur une image médicale de planification sur laquelle sont visibles à la fois la lésion et les éléments radio-opaques 222 du marqueur patient 22. Lorsque la position du marqueur patient 22 est connue dans le référentiel du système de navigation ou dans le référentiel du robot médical 10, il devient alors possible d'en déduire la position du point d'entrée et la position du point cible dans le référentiel du système de navigation ou dans le référentiel du robot médical 10.

Lorsque la position de la sonde 40 à ultrasons est connue à un instant donné, il est possible de déterminer la position d'un élément visible sur une image échographique acquise par la sonde à ultrasons 40 audit instant. Cet élément visible peut correspondre notamment au point cible ou au point d'entrée. Le point cible et le point d'entrée définissent une trajectoire que doit suivre l'instrument médical 15. Lorsque la position du point cible et la position du point d'entrée sont connues, c'est-à-dire lorsque la trajectoire que doit suivre l'instrument médical 15 est définie, l'unité de contrôle peut déplacer automatiquement le bras robotisé 13 dans une configuration permettant au guide-outil 14 de guider l'instrument médical 15 selon la trajectoire définie.

Les mouvements liés à la respiration du patient entrainent un déplacement du point cible et du point d'entrée dans le référentiel du robot médical 10. Ainsi, la trajectoire que doit suivre l'instrument médical 15 à un instant donné du cycle respiratoire du patient n'est pas la même à un autre instant du cycle respiratoire. Il convient donc de suivre en temps réel la position du point cible et la position du point d'entrée pour pouvoir à chaque instant déterminer la trajectoire que doit suivre l'instrument médical 15 et ajuster la position du guide-outil 14 de telle sorte qu'il guide l'instrument médical 15 selon cette trajectoire.

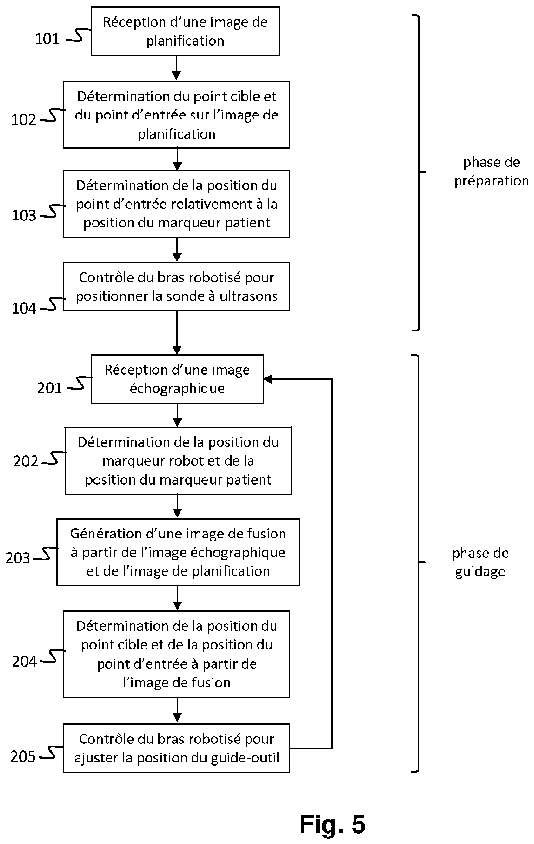

La figure 5 représente, à titre d'exemple, des étapes mises en œuvre par l'unité de contrôle 12 pour permettre ce suivi en temps réel. Ces étapes peuvent notamment avoir lieu avant l'insertion de l'instrument médical 15. Dans un premier temps, une phase de préparation consiste principalement à placer la sonde à ultrasons 40 à une position adéquate pour acquérir des images échographiques de la lésion. Dans un deuxième temps, une phase de guidage consiste à contrôler le bras robotisé 13 à partir des images échographiques acquises par la sonde à ultrasons 40 pour ajuster en temps réel la position du guide-outil 14, de telle sorte que l'instrument médical 15 soit constamment positionné selon la trajectoire à suivre.

La phase de préparation comporte une étape de réception 101 d'une image de planification sur laquelle est visible la lésion et au moins un élément radio-opaque 222 du marqueur patient 22. L'image de planification est par exemple une image médicale pré-interventionnelle acquise juste avant l'intervention lorsque le patient 20 est installé sur la table d'intervention, à un moment où le marqueur patient 22 est positionné sur le patient 20 à proximité de l'anatomie d'intérêt. L'image de planification peut également être une image médicale préopératoire acquise quelques jours ou quelques semaines avant l'intervention et recalée avec une image pré-interventionnelle. L'image de planification est par exemple une image médicale de tomodensitométrie, de tomographie par émission de positons ou d'imagerie par résonance magnétique. La position du marqueur patient 22 peut être déterminée sur l'image de planification grâce au marqueur radio-opaque 222 du marqueur patient 22 qui est visible sur l'image de planification.

La phase de préparation comporte ensuite une étape de détermination 102 d'un point cible et un point d'entrée sur l'image de planification. Selon un premier exemple, l'image de planification est affichée sur un écran de l'interface utilisateur 19, et l'interface utilisateur 19 permet au praticien d'identifier sur l'image de planification un point cible au niveau de la région à traiter, et/ou un point d'entrée au niveau de la peau du patient, et/ou une zone à risque à éviter (par exemple les os ou les vaisseaux sanguins), ainsi que des paramètres de traitement. Cette étape peut être facilitée par une segmentation de certaines régions anatomiques (l'anatomie d'intérêt, la lésion à traiter, des zones à risque, etc.) par un algorithme d'apprentissage automatique. Selon un autre exemple, le point cible et le point d'entrée peuvent être directement déterminés sur l'image de planification par un algorithme d'intelligence artificielle.

La phase de préparation comporte ensuite une étape de détermination 103 de la position du point cible et de la position du point d'entrée relativement à la position du marqueur patient 22. La position du marqueur patient 22 peut en effet être déterminée sur l'image de planification grâce aux éléments radio-opaques 222 qui sont visibles sur l'image de planification. En outre, grâce au système de navigation 30, la position du marqueur patient 22 peut être déterminée à tout instant dans le référentiel du système de navigation 30 ou dans le référentiel du robot médical 10. Il est donc possible d'en déduire la position du point d'entrée et la position du point cible dans le référentiel du système de navigation ou dans le référentiel du robot médical 10.

La phase de préparation comporte enfin une étape de contrôle en temps réel du bras robotisé 13 pour positionner la sonde à ultrason 40 de telle sorte qu'elle soit au contact du patient 20 et qu'une image échographique acquise par la sonde à ultrason 40 à cette position soit dans un plan contenant la lésion et la trajectoire que doit suivre l'instrument médical 15. Pour rappel, le guide-outil 14 et la sonde à ultrasons 40 sont agencés l'un par rapport à l'autre de telle sorte que l'instrument médical 15 se trouve toujours dans le plan d'une image échographique acquise par la sonde à ultrasons 40. Le contrôle en temps réel du bras robotisé 13 inclut le contrôle du bras supplémentaire 16 auquel est fixé la sonde à ultrasons 40.

Dans l'exemple considéré, le capteur d'effort 17 permet à l'unité de contrôle 13 de déterminer une pression exercée par la sonde à ultrasons 40 sur le corps du patient 20. L'unité de contrôle 12 est configurée pour déplacer le bras robotisé 13 de sorte que la sonde à ultrasons 40 exerce une pression prédéterminée sur le corps du patient 20. De telles dispositions permettent de maintenir la sonde à ultrasons 40 au contact du corps du patient 20 pendant les mouvements respiratoires du patient 20. Lorsqu'un mouvement respiratoire du patient 20 induit une pression trop importante de la sonde à ultrasons 40 sur le corps du patient 20 (inspiration), alors la sonde à ultrasons 40 est déplacée dans une direction opposée à celle du corps du patient 20. Au contraire, lorsqu'un mouvement respiratoire du patient 20 induit une pression trop faible de la sonde à ultrasons 40 sur le corps du patient 20 (expiration), alors la sonde à ultrasons est déplacée en direction du corps du patient 20.

Le point cible et le point d'entrée initialement définis par le praticien sur l'image de planification peuvent ensuite être suivis pendant la phase de guidage sur des images échographiques acquises en temps réel par la sonde à ultrasons.

Le suivi du point cible peut notamment être mis en œuvre par une méthode de suivi de mouvement dans plusieurs images successives, par une analyse de déformation du « speckle » ou par un algorithme d'intelligence artificielle. Lorsque la lésion n'est pas visible sur l'image échographique, pour aider au suivi du point cible sur les images de fusion, il peut être avantageux de suivre le mouvement d'une structure anatomique proche de la lésion visible sur les images échographiques (par exemple un vaisseau sanguin). La structure anatomique choisie doit toutefois être visible dans le plan d'une image échographique acquise par la sonde à ultrasons.

Si la lésion n'est pas suffisamment visible sur les images échographiques, il convient de faire le suivi du mouvement du point cible sur des images de fusion, chaque image de fusion correspondant à un recalage de l'image échographique avec l'image de planification.

La phase de guidage comporte une étape de réception 201 d'une image échographique acquise par la sonde à ultrasons 40.

La phase de guidage comporte une étape de détermination 202 de la position du marqueur robot 18 et de la position du marqueur patient 22 à l'instant auquel l'image échographique a été acquise par la sonde à ultrasons 40.

La phase de guidage comporte ensuite une étape de génération 203 d'une image de fusion résultant d'un recalage de l'image échographique avec l'image de planification. La lésion, le point cible, le point d'entrée sont donc visibles sur l'image de fusion obtenue.

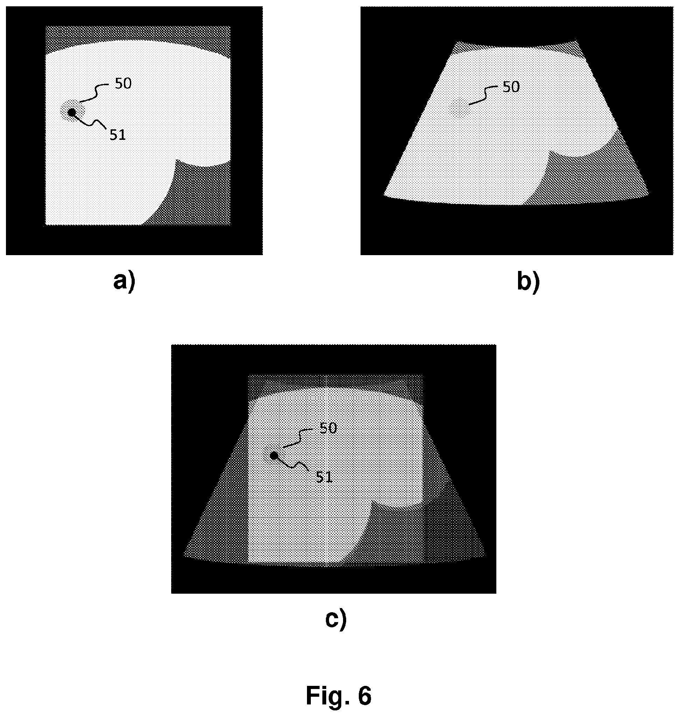

La figure 6 illustre schématiquement une image de planification (partie a) de la figure 6) pour recaler une image échographique (partie b) de la figure 6) afin de former une image de fusion (partie c) de la figure 6) résultant du recalage de l'image de planification avec l'image échographique. La lésion à traiter 50 et le point cible 51 sont visibles sur l'image de planification. Dans l'exemple considéré, l'image de référence est acquise par tomodensitométrie. La lésion à traiter 50 n'est en revanche quasiment pas visible sur l'image échographique. La lésion à traiter 50 et le point cible 51 deviennent visibles sur l'image d'analyse issue du recalage de l'image de référence avec l'image échographique. Il convient de noter que (même si cela n'est pas représenté sur la figure 6) les marqueurs radio-opaques 222 du marqueur patient 22 sont également visible sur l'image de planification et sur l'image de fusion.

Le recalage peut être global (recalage sur la totalité de l'anatomie d'intérêt) ou local (recalage optimisé sur une zone particulière de l'anatomie d'intérêt). Le recalage peut être effectué de manière rigide (par translation et/ou rotation) ou de manière non-rigide (avec déformation). Le recalage peut notamment être mis en œuvre par un algorithme d'apprentissage automatique basé sur la reconnaissance de structures anatomiques particulières sur les images à fusionner. Le recalage peut également être basé sur une segmentation de l'élément radio-opaque du marqueur patient sur l'image de planification puis par un recalage entre le référentiel de l'image de planification (connu via la position du marqueur patient 22) et le référentiel de l'image échographique (connu via la position du marqueur robot 18).

La phase de guidage comporte ensuite une étape de détermination 204 de la position du point cible et de la position du point d'entrée à partir de l'image de fusion, de la position du marqueur robot 18 et de la position du marqueur patient 22. La position du point cible et la position du point d'entrée peuvent être définie relativement à la position du marqueur patient 22 dans un référentiel de l'image de fusion. La connaissance de la position du marqueur patient 22 et de la position du marqueur robot 18 permet alors de déterminer la position du point cible et la position du point d'entrée dans le référentiel du système de navigation 30 et/ou dans le référentiel du robot médical 10.

Il convient toutefois de noter qu'il n'est pas indispensable de déterminer la position du marqueur robot 18 et la position du marqueur patient 22 pour chaque nouvelle image échographique acquise par la sonde à ultrasons 40 (cela signifie que l'étape 202 est optionnelle). En effet, comme la position de la sonde à ultrasons 40 est connue dans le référentiel du robot médical 10, le référentiel de l'image de fusion peut être défini par rapport au référentiel du robot médical 10, et il est donc possible de déterminer la position du point cible et la position du point d'entrée dans le référentiel du robot médical 10 directement à partir de l'image de fusion.

La phase de guidage comporte ensuite une étape de déplacement 205 du bras robotisé 13 de sorte que l'instrument médical 15 soit guidé par le guide-outil 14 selon la trajectoire définie par la position du point cible et la position du point d'entrée.

Les étapes 201 à 205 sont répétées pour chaque nouvelle image échographique reçue de la sonde à ultrasons 40.

Le bras robotisé est ainsi déplacé en temps réel pour qu'il soit en permanence positionné de telle sorte que l'instrument médical 15 soit guidé selon la trajectoire définie par la position du point cible et la position du point d'entrée. Cet ajustement en temps réel de la position du bras robotisé 13 permet de compenser le mouvement du point cible engendré par la respiration du patient.

Avec de telles dispositions, il devient possible de bloquer la respiration du patient à n'importe quel instant du cycle respiratoire pour procéder à l'insertion de l'instrument médical 15. En effet, quel que soit l'instant où la respiration du patient est bloquée, le bras robotisé 13 sera correctement positionné pour permettre l'insertion de l'instrument médical 15 selon la trajectoire souhaitée.

Aussi, il n'est même plus indispensable de bloquer la respiration du patient pendant l'intervention. En effet, le bras robotisé est déplacé en temps réel pour que la position du bras robotisé soit constamment ajustée pour guider l'instrument médical selon la trajectoire souhaitée.

Dès que l'instrument médical 15 commence à être inséré dans le corps du patient 20, la position du point d'entrée au niveau de la peau du patient est fixe et devient un pivot de rotation pour les mouvements du bras robotisé 13. Il reste toutefois possible de suivre en temps réel la position du point cible, la position du point d'entrée à partir de nouvelles images échographiques acquises en temps réel pendant l'insertion de l'instrument médical 15. Cela permet notamment de prendre en compte un éventuel déplacement du point cible résultant de l'insertion de l'instrument médical 15. Le point cible peut en effet se déplacer dans la direction de la trajectoire suivie par l'instrument médical 15 au cours de son insertion (c'est notamment le cas lorsque la lésion est située dans des tissus mous). La détermination en temps réel de la position du point cible à l'aide des images échographiques permettent de mettre à jour en temps réel la trajectoire que doit suivre l'instrument médical 15 ainsi que la position du bras robotisé 13 pour guider l'instrument médical 15 selon cette trajectoire.

Il convient de noter que la position de la sonde à ultrasons 40 peut être ajustée pendant la phase de guidage, en fonction de la position du marqueur patient 22 et/ou en fonction des mesures remontées par le capteur d'effort 17, pour rester au contact du patient 20 et pour rester dans un plan contenant la lésion et la trajectoire que doit suivre l'instrument médical 15. Les degrés de liberté additionnels apportés par le bras supplémentaire 16 permettent d'ajuster la position de la sonde à ultrasons 40 sans impacter la position du guide-outil 14.

Pendant la phase de préparation, si la lésion n'est pas dans le champ de vue de la sonde à ultrasons 40, c'est-à-dire si la lésion n'est pas visible sur une image échographique acquise par la sonde à ultrasons 40 (ou par l'image de fusion associée), il convient de déplacer la sonde à ultrasons 40 pour que pour que la lésion soit dans le champ de vue de la sonde à ultrasons 40. Dans ce but, l'unité de contrôle 12 peut être configurée pour comparer une image échographique avec l'image de planification (sur laquelle la lésion est visible) et pour déterminer une direction dans laquelle il convient de déplacer la sonde à ultrasons 40 pour qu'une image échographique acquise par la sonde à ultrasons 40 comporte une région anatomique dans laquelle se trouve la lésion. L'unité de contrôle 12 peut alors ensuite contrôler le bras robotisé 13 pour déplacer la sonde à ultrasons 40 dans cette direction. Alternativement, l'unité de contrôle 12 peut contrôler le bras robotisé 13 pour effectuer un balayage avec la sonde à ultrasons 40 jusqu'à ce que la lésion soit détectée sur une image échographique acquise par la sonde à ultrasons (ou sur l'image de fusion associée).

## Revendications

1. Robot médical (10) pour assister un praticien lors d'une intervention médicale pour traiter une lésion dans une anatomie d'intérêt d'un patient (20), ledit robot médical (10) comportant un bras robotisé (13) auquel est fixé à une extrémité une sonde à ultrasons (40) et un guide-outil (14) destiné à guider un instrument médical (15), ainsi qu'une unité de contrôle (12) configurée pour contrôler le bras robotisé (13), le robot médical (10) étant configuré pour coopérer avec un système de navigation (30), l'unité de contrôle (12) étant configurée pour pouvoir déterminer à tout instant, à partir d'informations communiquées par le système de navigation (30), la position d'un marqueur robot (18) destiné à être positionné sur le robot médical (10) et la position d'un marqueur patient (22) destiné à être positionné sur le patient (20) à proximité de l'anatomie d'intérêt,
pendant une phase de préparation, l'unité de contrôle (12) est configurée pour :
- recevoir une image de planification sur laquelle est visible la lésion et au moins un élément radio-opaque (222) du marqueur patient (22),
- déterminer à partir de l'image de planification un point cible au niveau de la lésion et un point d'entrée au niveau de la peau du patient (20), le point cible et le point d'entrée définissant ainsi une trajectoire à suivre pour l'instrument médical (15),
- contrôler le bras robotisé (13), en fonction de la position du marqueur robot (18) et de la position du marqueur patient (22), pour placer la sonde à ultrasons (40) au contact du patient (20) et dans un plan contenant la lésion et la trajectoire à suivre,
pendant une phase de guidage, l'unité de contrôle (12) est configurée pour recevoir en temps réel des images échographiques acquises par la sonde à ultrasons (40) et pour contrôler en temps réel le bras robotisé (13), à partir desdites images échographiques, afin de placer le guide-outil (14) de sorte à guider l'instrument médical (15) selon la trajectoire à suivre, l'unité de contrôle (12) étant
configurée pour comparer une image échographique avec l'image de planification, et pour déterminer une direction dans laquelle il convient de déplacer la sonde à ultrasons (40) pour qu'une image échographique acquise par la sonde à ultrasons (40) comporte une région anatomique dans laquelle se trouve la lésion.

2. Robot médical (10) selon la revendication 1 dans lequel, pendant la phase de guidage, l'unité de contrôle (12) est configurée, pour chaque image échographique reçue, pour :
- générer une image de fusion résultant d'un recalage de l'image échographique avec l'image de planification,
- déterminer la position du point cible et la position du point d'entrée à partir de l'image de fusion,
- déplacer le bras robotisé (13) de sorte que l'instrument médical (15) soit guidé par le guide-outil (14) selon la trajectoire définie par la position du point cible et la position du point d'entrée.

3. Robot médical (10) selon l'une des revendications 1 à 2 dans lequel pendant la phase de guidage, lors de l'insertion de l'instrument médical (15), pour chaque nouvelle image échographique reçue, l'unité de contrôle (12) est configurée pour déterminer la position de l'instrument médical (15) et pour ajuster le contrôle en temps réel du bras robotisé (13) en fonction de la position de l'instrument médical (15).

4. Robot médical (10) selon l'une des revendications 1 à 3 dans lequel la sonde à ultrasons (40) est couplée à un capteur d'effort (17) permettant à l'unité de contrôle (13) de déterminer une pression exercée par la sonde à ultrasons (40) sur le corps du patient (20), et l'unité de contrôle (12) est configurée pour déplacer le bras robotisé (13) de sorte que la sonde à ultrasons (40) exerce une pression prédéterminée sur le corps du patient (20).

5. Robot médical (10) selon l'une quelconque des revendications 1 à 4 dans lequel, l'image de planification est une image de tomodensitométrie, une image de tomographie par émission de positons ou une image d'imagerie par résonance magnétique.

6. Robot médical (10) selon l'une quelconque des revendications 1 à 5 dans lequel, pour déterminer à partir de l'image de planification un point cible et un point d'entrée, l'unité de contrôle est configurée pour segmenter sur l'image de planification la lésion et/ou des zones anatomiques à éviter à l'aide d'un algorithme d'intelligence artificielle.

7. Robot médical (10) selon l'une quelconque des revendications 1 à 6 dans lequel les images échographiques reçues de la sonde à ultrasons (40) sont des images échographiques en mode B.

8. Robot médical (10) selon l'une quelconque des revendications 1 à 7 dans lequel l'unité de contrôle (12) est configurée pour recevoir et traiter des images échographiques acquises par la sonde à ultrasons (40) à une fréquence au moins égale à quinze images par seconde.

9. Robot médical (10) selon l'une quelconque des revendications 1 à 8 comportant une interface utilisateur (19) comprenant un écran d'affichage permettant au praticien de visualiser l'image de planification et/ou les images de fusion.

10. Robot médical (10) selon la revendication 9 dans lequel l'interface utilisateur (19) comporte des moyens d'entrée permettant au praticien, pendant la phase de préparation, d'identifier sur l'image de planification affichée sur l'écran d'affichage un point cible et/ou un point d'entrée et/ou une zone anatomique qui ne doit pas être traversée par l'instrument médical (15).

11. Robot médical (10) selon l'une quelconque des revendications 9 à 10 dans lequel l'interface utilisateur comprend un dispositif de réalité augmentée permettant de superposer les images d'analyse avec des images réelles du corps du patient sur l'écran d'affichage.

## Patentansprüche

1. Medizinischer Roboter (10) zur Unterstützung eines Arztes bei einem medizinischen Eingriff zur Behandlung einer Läsion in einer Anatomie von Interesse eines Patienten (20), wobei der medizinische Roboter (10) einen Roboterarm (13), an dem an einem Ende eine Ultraschallsonde (40) und eine Werkzeugführung (14) zur Führung eines medizinischen Instruments (15) befestigt sind, sowie eine Steuereinheit (12), die zur Steuerung des Roboterarms (13) eingerichtet ist, aufweist, wobei der medizinische Roboter (10) eingerichtet ist, um mit einem Navigationssystem (30) zusammenzuwirken, wobei die Steuereinheit (12) so eingerichtet ist, dass sie jederzeit aus Informationen, die von dem Navigationssystem (30) übermittelt werden, die Position eines Robotermarkers (18), der auf dem medizinischen Roboter (10) positioniert werden soll, und die Position eines Patientenmarkers (22), der auf dem Patienten (20) in der Nähe der betreffenden Anatomie positioniert werden soll, bestimmen kann,
während einer Vorbereitungsphase ist die Steuereinheit (12) zu Folgendem eingerichtet:
- Empfangen eines Planungsbilds, auf dem die Läsion sichtbar ist, und mindestens eines röntgendichten Elements (222) des Patientenmarkers (22),
- Bestimmen eines Zielpunkts auf Höhe der Läsion und eines Eintrittspunkts auf Höhe der Haut des Patienten (20) aus dem Planungsbild, wobei der Zielpunkt und der Eintrittspunkt somit einen zu verfolgenden Pfad für das medizinische Instrument (15) definieren,
- Steuern des Roboterarms (13) in Abhängigkeit von der Position des Robotermarkers (18) und der Position des Patientenmarkers (22), um die Ultraschallsonde (40) in Kontakt mit dem Patienten (20) und in einer Ebene zu platzieren, die die Läsion und den zu verfolgenden Pfad enthält,
während einer Führungsphase ist die Steuereinheit (12) so eingerichtet, dass sie in Echtzeit von der Ultraschallsonde (40) erfasste Ultraschallbilder empfängt und den Roboterarm (13) ausgehend von den Ultraschallbildern in Echtzeit steuert, um die Werkzeugführung (14) so zu positionieren, dass sie das medizinische Instrument (15) entlang des zu verfolgenden Pfads führt,
wobei die Steuereinheit (12) so eingerichtet ist, dass sie ein Ultraschallbild mit dem Planungsbild vergleicht und eine Richtung bestimmt, in die die Ultraschallsonde (40) bewegt werden soll, damit ein von der Ultraschallsonde (40) erfasstes Ultraschallbild einen anatomischen Bereich aufweist, in dem sich die Läsion befindet.

2. Medizinischer Roboter (10) nach Anspruch 1, wobei die Steuereinheit (12) während der Führungsphase für jedes empfangene Ultraschallbild zu Folgendem eingerichtet ist:
- Erzeugen eines Fusionsbildes, das aus einer Neukalibrierung des Ultraschallbildes mit dem Planungsbild resultiert,
- Bestimmen der Position des Zielpunktes und der Position des Eintrittspunktes aus dem Fusionsbild,
- Bewegen des Roboterarms (13) so, dass das medizinische Instrument (15) durch die Werkzeugführung (14) entlang der durch die Position des Zielpunkts und die Position des Eintrittspunkts definierten Bahn geführt wird.

3. Medizinischer Roboter (10) nach einem der Ansprüche 1 bis 2, wobei während der Führungsphase bei dem Einführen des medizinischen Instruments (15) für jedes neue empfangene Ultraschallbild die Steuereinheit (12) so eingerichtet ist, dass sie die Position des medizinischen Instruments (15) bestimmt und die Echtzeitsteuerung des Roboterarms (13) in Abhängigkeit von der Position des medizinischen Instruments (15) anpasst.

4. Medizinischer Roboter (10) nach einem der Ansprüche 1 bis 3, wobei die Ultraschallsonde (40) mit einem Kraftsensor (17) gekoppelt ist, der es der Steuereinheit (13) ermöglicht, einen Druck zu ermitteln, der von der Ultraschallsonde (40) auf den Körper des Patienten (20) ausgeübt wird, und die Steuereinheit (12) so eingerichtet ist, dass sie den Roboterarm (13) so bewegt, dass die Ultraschallsonde (40) einen vorbestimmten Druck auf den Körper des Patienten (20) ausübt.

5. Medizinischer Roboter (10) nach einem der Ansprüche 1 bis 4, wobei das Planungsbild ein CT-Bild, ein Positronen-Emissions-Tomographie-Bild oder ein Magnetresonanz-Bild ist.

6. Medizinischer Roboter (10) nach einem der Ansprüche 1 bis 5, wobei die Steuereinheit zum Bestimmen eines Zielpunkts und eines Eintrittspunkts aus dem Planungsbild so eingerichtet ist, dass sie die Läsion und/oder die zu vermeidenden anatomischen Bereiche mithilfe eines künstlichen Intelligenzalgorithmus auf dem Planungsbild segmentiert.

7. Medizinischer Roboter (10) nach einem der Ansprüche 1 bis 6, wobei die von der Ultraschallsonde (40) empfangenen Ultraschallbilder Ultraschallbilder in dem Modus B sind.

8. Medizinischer Roboter (10) nach einem der Ansprüche 1 bis 7, wobei die Steuereinheit (12) so eingerichtet ist, dass sie von der Ultraschallsonde (40) erfasste Ultraschallbilder mit einer Frequenz von mindestens fünfzehn Bildern pro Sekunde empfängt und verarbeitet.

9. Medizinischer Roboter (10) nach einem der Ansprüche 1 bis 8, der eine Benutzeroberfläche (19) aufweist, die einen Anzeigebildschirm umfasst, mit dem der Arzt das Planungsbild und/oder die Fusionsbilder visualisieren kann.

10. Medizinischer Roboter (10) nach Anspruch 9, wobei die Benutzerschnittstelle (19) Eingabemittel aufweist, die es dem Arzt ermöglichen, während der Vorbereitungsphase auf dem auf dem Anzeigebildschirm angezeigten Planungsbild einen Zielpunkt und/oder einen Eintrittspunkt und/oder einen anatomischen Bereich zu identifizieren, der nicht von dem medizinischen Instrument (15) durchquert werden soll.

11. Medizinischer Roboter (10) nach einem der Ansprüche 9 bis 10, wobei die Benutzeroberfläche eine Augmented-Reality-Vorrichtung umfasst, die es ermöglicht, die Analysebilder mit echten Bildern des Körpers des Patienten auf dem Anzeigebildschirm zu überlagern.

## Claims

1. Medical robot (10) for assisting a practitioner during a medical procedure to treat a lesion in an anatomy of interest of a patient (20), said medical robot (10) comprising a robotic arm (13) to which an ultrasonic probe (40) is fastened at one end and a tool guide (14) intended to guide a medical instrument (15), as well as a control unit (12) configured to control the robotic arm (13), the medical robot (10) being configured to cooperate with a navigation system (30), the control unit (12) being configured to be able to determine at any time, from information communicated by the navigation system (30), the position of a robot marker (18) intended to be positioned on the medical robot (10) and the position of a patient marker (22) intended to be positioned on the patient (20) near the anatomy of interest,
during a preparation phase, the control unit (12) is configured to:
- receive a planning image on which the lesion is visible and at least one radiopaque element (222) of the patient marker (22),
- determine from the planning image a target point at the lesion and an entry point at the skin of the patient (20), the target point and the entry point thus defining a trajectory to be followed for the medical instrument (15),
- control the robotic arm (13), according to the position of the robot marker (18) and the position of the patient marker (22), to place the ultrasonic probe (40) in contact with the patient (20) and in a plane containing the lesion and the trajectory to be followed,
during a guide phase, the control unit (12) is configured to receive in real time ultrasound images acquired by the ultrasonic probe (40) and to control in real time the robotic arm (13), on the basis of said ultrasound images, in order to place the tool guide (14) so as to guide the medical instrument (15) according to the trajectory to be followed,
the control unit (12) being configured to compare an ultrasound image to the planning image, and to determine a direction in which the ultrasonic probe (40) should be moved so that an ultrasound image acquired by the ultrasonic probe (40) comprises an anatomical region in which the lesion is located.

2. Medical robot (10) according to claim 1, wherein during the guide phase, the control unit (12) is configured, for each ultrasound image received, to:
- generate a fusion image resulting from a registration of the ultrasound image with the planning image,
- determine the position of the target point and the position of the entry point from the fusion image,
- move the robotic arm (13) so that the medical instrument (15) is guided by the tool guide (14) according to the trajectory defined by the position of the target point and the position of the entry point.

3. Medical robot (10) according to one of claims 1 to 2, wherein during the guide phase, during the insertion of the medical instrument (15), for each new ultrasound image received, the control unit (12) is configured to determine the position of the medical instrument (15) and to adjust the real-time control of the robotic arm (13) according to the position of the medical instrument (15).

4. Medical robot (10) according to one of claims 1 to 3, wherein the ultrasonic probe (40) is coupled to a force sensor (17) allowing the control unit (13) to determine a pressure exerted by the ultrasonic probe (40) on the body of the patient (20), and the control unit (12) is configured to move the robotic arm (13) so that the ultrasonic probe (40) exerts a predetermined pressure on the body of the patient (20).

5. Medical robot (10) according to any one of claims 1 to 4, wherein the planning image is a computed tomography image, a positron emission tomography image or a magnetic resonance imaging image.

6. Medical robot (10) according to any one of claims 1 to 5, wherein to determine from the planning image a target point and an entry point, the control unit is configured to segment on the planning image the lesion and/or anatomical areas to be avoided using an artificial intelligence algorithm.

7. Medical robot (10) according to any one of claims 1 to 6, wherein the ultrasound images received from the ultrasonic probe (40) are B-mode ultrasound images.

8. Medical robot (10) according to any one of claims 1 to 7, wherein the control unit (12) is configured to receive and process ultrasound images acquired by the ultrasonic probe (40) at a frequency at least equal to fifteen images per second.

9. Medical robot (10) according to any one of claims 1 to 8, comprising a user interface (19) comprising a display screen allowing the practitioner to view the planning image and/or the fusion images.

10. Medical robot (10) according to claim 9, wherein the user interface (19) comprises input means allowing the practitioner, during the preparation phase, to identify on the planning image displayed on the display screen a target point and/or an entry point and/or an anatomical area through which the medical instrument (15) must not pass.

11. Medical robot (10) according to any one of claims 9 to 10, wherein the user interface comprises an augmented reality device allowing to superimpose the analysis images with real images of the body of the patient on the display screen.
